(19) Europäisches Patentamt
European Patent Office
Office européen des brevets

(11) Veröffentlichungsnummer : **0 438 726 B1**

(12) # EUROPÄISCHE PATENTSCHRIFT

(45) Veröffentlichungstag der Patentschrift :
**16.03.94 Patentblatt 94/11**

(51) Int. Cl.$^5$ : **C07C 323/61,** A01N 37/36

(21) Anmeldenummer : **90124481.4**

(22) Anmeldetag : **18.12.90**

(54) **Ungesättigte Cyclohexylessigsäurederivate und diese enthaltende Pflanzenschutzmittel.**

(30) Priorität : **20.01.90 DE 4001618**

(43) Veröffentlichungstag der Anmeldung :
**31.07.91 Patentblatt 91/31**

(45) Bekanntmachung des Hinweises auf die Patenterteilung :
**16.03.94 Patentblatt 94/11**

(84) Benannte Vertragsstaaten :
**AT BE CH DE DK ES FR GB GR IT LI NL SE**

(56) Entgegenhaltungen :
**EP-A- 0 370 629**
**DE-A- 3 843 439**
**US-A- 4 299 969**

(73) Patentinhaber : **BASF Aktiengesellschaft**
**Carl-Bosch-Strasse 38**
**D-67063 Ludwigshafen (DE)**

(72) Erfinder : **Mueller, Bernd, Dr.**
**Jean-Ganss-Strasse 21**
**W-6710 Frankenthal (DE)**
Erfinder : **Sauter, Hubert, Dr.**
**Neckarpromenade 20**
**W-6800 Mannheim 1 (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 3**
**W-6730 Neustadt (DE)**

**EP 0 438 726 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Cyclohexylessigsäurederivate mit fungizider und insektizider Wirkung, diese Verbindungen enthaltende Fungizide und Insektizide und Verfahren zur Bekämpfung von Pilzen.

Es wurde gefunden, daß ungesättigte Cyclohexylessigsäurederivate der Formel I

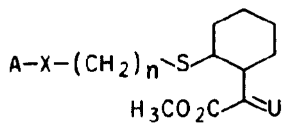

I

in der U $=NOCH_3$, $=CHOCH_3$, $=CH_2$, $=CH-CH_3$, $=CH-CH_2-CH_3$, $=N-NH-CH_3$ oder $=CH-S-CH_3$ bedeutet,
n die Zahlen 0 bis 10 bedeutet,
A Wasserstoff oder die gegebenenfalls substituierten Reste Alkyl, Cycloalkyl, Aryl oder Hetaryl bedeutet und
X im Fall n=O eine Einfachbindung und im Fall n verschieden von 0 Sauerstoff oder Schwefel oder eine Einfachbindung bedeutet, sowie deren pflanzenverträgliche Säureadditionsprodukte und Basenadditionsprodukte neben einer hohen fungitoxischen und insektiziden Wirkung auch eine sehr gute Pflanzenverträglichkeit besitzen.

Säuren für Säureadditionsprodukte sind z.B. Mineralsäuren, wie beispielsweise Chlorwasserstoffsäure, Bromwasserstoffsäure, Phosphorsäure, Schwefelsäure, Salpetersäure, oder aber Carbonsäuren, wie Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure, oder aber auch allgemein Protonen-acide Verbindungen, z.B. Saccharin.

Basen für Basenadditionsprodukte sind z.B. Kalium-, Natrium-, -hydroxid, -carbonat, Ammoniumhydroxid.

Die neuen Verbindungen der Formel I können bei der Herstellung als Gemische von Stereoisomeren (E/Z-Isomere, Diastereomere, Enantiomere) anfallen, die in üblicher Weise, z.B. durch Kristallisation oder Chromatographie, in die Einzelkomponenten getrennt werden können. Sowohl die einzelnen Isomeren als auch ihre Gemische können als Fungizide oder Insektizide verwendet werden und werden von der Erfindung erfaßt.

Falls U in syn/anti-Isomeren vorkommen kann, werden von der Erfindung alle Isomeren, insbesondere die anti-Isomeren umfaßt.

Besonders werden alle Diastereomeren erfaßt, insbesondere diejenigen mit bis-equatorialer Anordnung an den beiden Chiralitätszentren im Cyclohexanring.

U

bedeutet $=NOCH_3$, $=CHOCH_3$, $=CH_2$, $=CH-CH_3$, $=CH-CH_2-CH_3$, $=N-NH-CH_3$, $=CH-S-CH_3$, insbesondere =O, $=NOCH_3$, $=CH-OCH_3$, $=CH-CH_3$, vorzugsweise $=NOCH_3$

n

bedeutet 0 bis 10, insbesondere 0 bis 5, vorzugsweise 0, 1, 2, 3.

A

bedeutet beispielsweise einen gegebenenfalls ungesättigten geradkettigen oder verzweigten Kohlenwasserstoffrest mit 1 bis 6, insbesondere 1 bis 4 C-Atomen oder einen $C_3$-$C_6$-Cycloalkylrest oder Phenyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, wobei die genannten Cycloalkyle, Alkyle, Aryle oder Hetaryle, gegebenenfalls durch 1 bis 3 gleiche oder verschiedene Substituenten aus der Gruppe Halogen, Fluor, Chlor, Brom, Jod, $C_1$-$C_8$-Alkyl, $C_1$-$C_8$-Alkylthio, $C_1$-$C_8$-Alkylsulfinyl, Cyano, Hydroxy, Nitro, $C_1$-$C_8$-Alkoximino, $C_1$-$C_8$-Alkoxy, Phenyl, $C_1$-$C_4$-Halogenalkyl, $C_1$-$C_8$-Alkenyloximino, Formyl, $C_1$-$C_9$-Alkylcarbonyl oder $C_1$-$C_8$-Alkoxycarbonyl oder Phenyl substituiert durch 1 bis 3 gleiche oder verschiedene Substituenten aus der Gruppe Halogen, Fluor, Chlor, Brom, Iod, $C_1$-$C_8$-Alkyl, $C_1$-$C_8$-Alkylthio, $C_1$-$C_8$-Alkylsulfinyl, Cyano, Hydroxy, Nitro, $C_1$-$C_8$-Alkoximino, $C_1$-$C_8$-Alkoxy, Phenyl, $C_1$-$C_4$-Halogenalkyl, $C_1$-$C_8$-Alkenyloximino, Formyl, $C_1$-$C_9$-Alkylcarbonyl substituiert sind.

A

bedeutet vorzugsweise einen gegebenenfalls ungesättigten geradkettigen oder verzweigten Kohlenwasserstoffrest mit 1 bis 6, insbesondere 1 bis 4 C-Atomen oder einen $C_3$-$C_6$-Cycloalkylrest oder Phenyl, Pyridyl oder Benzimidazolyl.

Die neuen Verbindungen können beispielsweise nach folgenden Verfahren hergestellt werden:
α-Cyclohexen-1-yl-α-oxo-essigsäuremethylester 1 (G. Neef et al., THL 1977 (32), 2825-8) wird mit Thiolen 2 ggf. unter Basenkatalyse zu den Thioethern 3 (Y. Vankar et al., J. Chem. Research 1989, 178-179) umgesetzt (Schema 1).

Schema 1

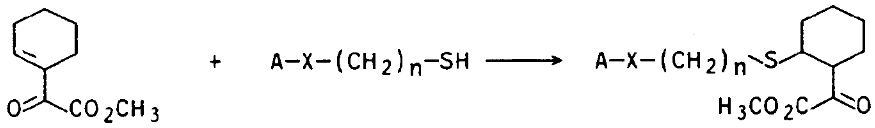

1 2 3

A, n, siehe oben.

Die substituierten α-Oxo-essigsäuremethylester 3 werden durch Reaktion mit $CH_3$-O-$NH_3$Cl bzw. $H_2$N-NH-$CH_3$ in die Wirkstoffe 4 überführt (Schema 2).

Schema 2

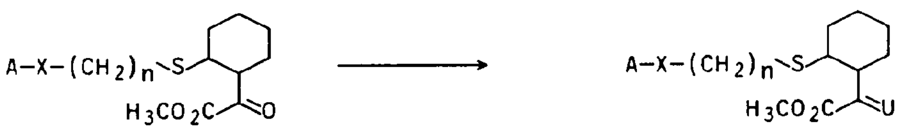

3 4

U: =$NOCH_3$, =N-$NHCH_3$

A, n, siehe oben.

Durch Wittig-Reaktion mit $(C_6H_5)_3P^+$-$CH_3X^-$, $(C_6H_5)_3P^+$-$CH_2$-$CH_3X^-$, $(C_6H_5)_3P^+$-$CH_2$-$CH_2$-$CH_3X^-$ (X=Halogen) bzw. $(C_6H_5)_3P^+$-$CH_2$-O-$CH_3Cl^-$, $(C_6H_5)_2P$(=O)-$CH_2$-O-$CH_3$ oder Peterson-Olefinierung mit $(CH_3)_3Si$-$CH_2$-O-$CH_3$ können aus den α-Oxo-essigsäuremethylestern 3 die Verbindungen 5 hergestellt werden (Schema 3).

Schema 3

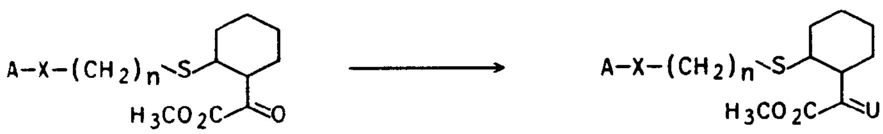

3 5

U: = $CH_2$, =CH-$CH_3$, =CH-$CH_2$-$CH_3$, =$CHOCH_3$

A, n, siehe oben.

Die Thioenolether 7 sind aus den Enolethern 6 durch Reaktion mit Methylmercaptan erhältlich (EP 178 826; Schema 4).

Schema 4

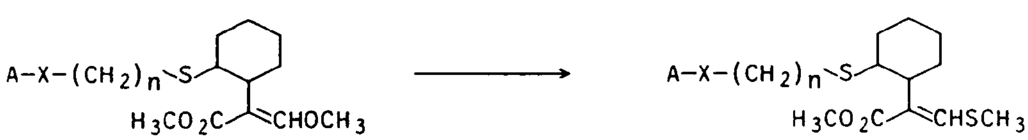

6 7

A, n, siehe oben.

Die folgenden Beispiele erläutern die Herstellung der neuen Verbindungen.

Beispiel 1

α-(2-Benzylthio-cyclohexyl)-α-oxo-essigsäuremethylester (Verbindung Nr. 1.15)

2 g (12 mmol) α-Cyclohexenyl-α-oxo-essigsäuremethylester, 1,5 g (12 mmol) Benzylmercaptan und 1 g

(10 mmol) Triethylamin in 10 ml Methylenchlorid werden 15 Stunden bei Raumtemperatur (20°C) gerührt.

Man versetzt den Reaktionsansatz nach dem Rühren mit Wasser und extrahiert die wässrige Phase mit Methylenchlorid. Die organische Phase wird mit Wasser extrahiert, über $MgSO_4$ getrocknet und eingeengt.

Nach chromatographischer Reinigung des Rohproduktes erhält man 2,3 g (66 %) der Titelverbindung als hellgelbes Öl.
IR ($cm^{-1}$): 2933, 1727, 1277, 1069

Beispiel 2

α-(2-Benzylthio-cyclohexyl-α-oxo-essigsäuremethylester-O-methyloxim (Verbindung Nr. 2.15)

2,3 g (7,9 mmol) α-(2-Benzylthio-cyclohexyl)-α-oxo-essigsäuremethylester (Beispiel 1) und 0,66 g (8 mmol) Methoxyaminhydrochlorid in 15 ml Methanol werden 15 Stunden bei Raumtemperatur gerührt.

Man versetzt die Reaktionsmischung mit Wasser und extrahiert die wässrige Phase mit Ether. Die organische Phase wird mit Wasser gewaschen, über $MgSO_4$ getrocknet und eingedampft. Nach chromatographischer Reinigung des Rohproduktes erhält man 1 g (40 %) der Titelverbindung als hellgelbes Öl. IR ($cm^{-1}$): 2935, 1726, 1151, 1042

Tabelle 1

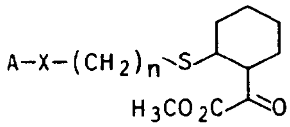
.

| Nr. | $A-X-(CH_2)_n$ | Fp. | IR: $\nu(cm^{-1})$ |
|---|---|---|---|
| 1. 1 | H | | |
| 1. 2 | Methyl | | |
| 1. 3 | Ethyl | | |
| 1. 4 | Isopropyl | | |
| 1. 5 | Butyl | | |
| 1. 6 | sec.-Butyl | | |
| 1. 7 | t-Butyl | | |
| 1. 8 | Isobutyl | | |
| 1. 9 | Pentyl | | |
| 1.10 | Pivaloyl | | |
| 1.11 | Hexyl | | |
| 1.12 | Octyl | | |
| 1.13 | Decyl | | |
| 1.14 | Phenyl | Öl | 2935, 1729, 1276, 1069 |
| 1.15 | Benzyl | Öl | 2933, 1727, 1277, 1069 |
| 1.16 | Phenylethyl | | |
| 1.17 | Phenylpropyl | | |
| 1.18 | Phenylpentyl | | |
| 1.19 | 1-Naphthyl | | |
| 1.20 | 2-Naphthyl | | |
| 1.21 | 2-Bromphenyl | | |
| 1.22 | 3-Bromphenyl | | |
| 1.23 | 4-Bromphenyl | | |
| 1.24 | 2-Chlorphenyl | | |
| 1.25 | 3-Chlorphenyl | | |
| 1.26 | 4-Chlorphenyl | | |
| 1.27 | 2-Fluorphenyl | | |
| 1.28 | 3-Fluorphenyl | | |
| 1.29 | 4-Fluorphenyl | | |
| 1.30 | 2-Methylphenyl | | |
| 1.31 | 3-Methylphenyl | | |
| 1.32 | 4-Methylphenyl | | |
| 1.33 | 2-Ethyl-phenyl | | |
| 1.34 | 3-Ethyl-phenyl | | |
| 1.35 | 4-Ethyl-phenyl | | |

Tabelle 1 (Fortsetzung)

| Nr. | A-X-$(CH_2)_n$ | Fp. | IR: $\nu(cm^{-1})$ |
|---|---|---|---|
| 1.36 | 2-iso-Propyl-phenyl | | |
| 1.37 | 3-iso-Propyl-phenyl | | |
| 1.38 | 4-iso-Propyl-phenyl | | |
| 1.39 | 2-tert.-Butyl-phenyl | | |
| 1.40 | 3-tert.-Butyl-phenyl | | |
| 1.41 | 4-tert.-Butyl-phenyl | | |
| 1.42 | 4-Butyl-phenyl | | |
| 1.43 | 4-Hexyl-phenyl | | |
| 1.44 | 4-Nonyl-phenyl | | |
| 1.45 | 4-Decyl-phenyl | | |
| 1.46 | 2-Methoxy-phenyl | | |
| 1.47 | 3-Methoxy-phenyl | | |
| 1.48 | 4-Methoxy-phenyl | | |
| 1.49 | 2-Trifluormethyl-phenyl | | |
| 1.50 | 3-Trifluormethyl-phenyl | | |
| 1.51 | 4-Trifluormethyl-phenyl | | |
| 1.52 | 2-Formylphenyl | | |
| 1.53 | 3-Formylphenyl | | |
| 1.54 | 4-Formylphenyl | | |
| 1.55 | 2-Allyl-O-N=CH-phenyl | | |
| 1.56 | 3-Allyl-O-N=CH-phenyl | | |
| 1.57 | 4-Allyl-O-N-CH-phenyl | | |
| 1.58 | 2-Nitro-phenyl | | |
| 1.59 | 3-Nitro-phenyl | | |
| 1.60 | 2,4-Dichlorphenyl | | |
| 1.61 | 2,5-Dichlor-phenyl | | |
| 1.62 | 2,6-Dichlor-phenyl | | |
| 1.63 | 3,4-Dichlor-phenyl | | |
| 1.64 | 2,3-Dichlor-phenyl | | |
| 1.65 | 3,5-Dichlor-phenyl | | |
| 1.66 | 2,3,4-Trichlor-phenyl | | |
| 1.67 | 2,4,5-Trichlor-phenyl | | |
| 1.68 | 2,4,6-Trichlor-phenyl | | |
| 1. 69 | 2,3,4,6-Tetrachlor-phenyl | | |
| 1. 70 | 2,3,4,5,6-Pentachlor-phenyl | | |
| 1. 71 | 2,3,4,5-Tetrafluor-phenyl | | |
| 1. 72 | 2,3,5,6-Tetrafluor-phenyl | | |
| 1. 73 | 2,3,4,5,6-Pentafluor-phenyl | | |

Tabelle 1 (Fortsetzung)

| Nr. | $A-X-(CH_2)_n$ | Fp. | IR: $\nu(cm^{-1})$ |
|---|---|---|---|
| 1. 74 | 2-Chlor, 4-Fluor-phenyl | | |
| 1. 75 | 3-Chlor, 4-Fluor-phenyl | | |
| 1. 76 | 2-Chlor, 6-Methyl-phenyl | | |
| 1. 77 | 4-Chlor, 2-Methyl-phenyl | | |
| 1. 78 | 2,4-Dichlor, 5-Methyl-phenyl | | |
| 1. 79 | 4-Chlor, 2,5-Dimethyl-phenyl | | |
| 1. 80 | 4-Brom, 2-Methyl-phenyl | | |
| 1. 81 | 3,5-Bistrifluormethyl-phenyl | | |
| 1. 82 | 2,3-Dimethyl-phenyl | | |
| 1. 83 | 2,4-Dimethyl-phenyl | | |
| 1. 84 | 2,5-Dimethyl-phenyl | | |
| 1. 85 | 2,6-Dimethyl-phenyl | | |
| 1. 86 | 3,4-Dimethyl-phenyl | | |
| 1. 87 | 3,5-Dimethyl-phenyl | | |
| 1. 88 | 2,4,5-Trimethyl-phenyl | | |
| 1. 89 | 2,6-Diethyl-phenyl | | |
| 1. 90 | 2,4-Di-tert.-Butyl-phenyl | | |
| 1. 91 | 2,5-Dimethoxy-phenyl | | |
| 1. 92 | 3,4-Dimethoxy-phenyl | | |
| 1. 93 | 2-Methyl, 4-tert.-Butyl-phenyl | | |
| 1. 94 | 2-Methoxycarbonyl-phenyl | | |
| 1. 95 | 2-Ethoxycarbonyl-phenyl | | |
| 1. 96 | 2-Propoxycarbonyl-phenyl | | |
| 1. 97 | 2-Butoxycarbonyl-phenyl | | |
| 1. 98 | 2-Cyano-phenyl | | |
| 1. 99 | 3-Cyano-phenyl | | |
| 1.100 | 4-Cyano-phenyl | | |
| 1.101 | 1-Naphthylmethyl | | |
| 1.102 | 2-Naphthylmethyl | | |
| 1.103 | 2-Brombenzyl | | |
| 1.104 | 3-Brombenzyl | | |
| 1.105 | 4-Brombenzyl | | |
| 1.106 | 2-Chlorbenzyl | | |
| 1.107 | 3-Chlorbenzyl | | |
| 1.108 | 4-Chlorbenzyl | | |
| 1.109 | 2-Fluorbenzyl | | |
| 1.110 | 3-Fluorbenzyl | | |
| 1.111 | 4-Fluorbenzyl | | |
| 1.112 | 2-Methylbenzyl | | |

Tabelle 1 (Fortsetzung)

| Nr. | A-X-$(CH_2)_n$ | Fp. | IR: $\nu(cm^{-1})$ |
|---|---|---|---|
| 1.113 | 3-Methylbenzyl | | |
| 1.114 | 4-Methylbenzyl | | |
| 1.115 | 2-Methoxybenyzl | | |
| 1.116 | 3-Methoxybenzyl | | |
| 1.117 | 4-Methoxybenzyl | | |
| 1.118 | 4-Ethoxybenzyl | | |
| 1.119 | 4-Butyloxybenzyl | | |
| 1.120 | 4-Benzyloxybenzyl | | |
| 1.121 | 2-Trifluormethyl-benzyl | | |
| 1.122 | 3-Trifluormethyl-benzyl | | |
| 1.123 | 4-Trifluormethyl-benzyl | | |
| 1.124 | 2-Formylbenzyl | | |
| 1.125 | 3-Formylbenzyl | | |
| 1.126 | 4-Formylbenzyl | | |
| 1.127 | 2-Allyl-O-N=CH-benzyl | | |
| 1.128 | 3-Allyl-O-N=CH-benzyl | | |
| 1.129 | 4-Allyl-O-N=CH-benzyl | | |
| 1.130 | 2-Nitrobenzyl | | |
| 1.131 | 3-Nitrobenzyl | | |
| 1.132 | 2,4-Dichlorbenzyl | | |
| 1.133 | 2,6-Dichlorbenzyl | | |
| 1.134 | 3,4-Dichlorbenzyl | | |
| 1.135 | 2,3-Dichlorbenzyl | | |
| 1.136 | 3,5-Dichlorbenzyl | | |
| 1.137 | 2,3-Dimethylbenzyl | | |
| 1.138 | 2,4-Dimethylbenzyl | | |
| 1.139 | 2,5-Dimethylbenzyl | | |
| 1.140 | 2,6-Dimethylbenzyl | | |
| 1.141 | 3,4-Dimethylbenzyl | | |
| 1.142 | 3,5-Dimethylbenzyl | | |
| 1.143 | 4-Chlor-2-methylbenzyl | | |
| 1.144 | 2-Methoxycarbonyl-benzyl | | |
| 1.145 | 2-Ethoxycarbonyl-benzyl | | |
| 1.146 | 2-Butoxycarbonyl-benzyl | | |
| 1.147 | 2-Cyano-benzyl | | |
| 1.148 | 3-Cyano-benzyl | | |
| 1.149 | 4-Cyano-benzyl | | |
| 1.150 | 2-Pyridyl | | |
| 1.151 | 3-Pyridyl | | |

Tabelle 1 (Fortsetzung)

| Nr. | $A-X-(CH_2)_n$ | Fp. | IR: $\nu(cm^{-1})$ |
|---|---|---|---|
| 1.152 | 4-Pyridyl | | |
| 1.153 | 6-Methyl-2-pyridyl | | |
| 1.154 | 6-Ethyl-2-pyridyl | | |
| 1.155 | 6-n-Propyl-2-pyridyl | | |
| 1.156 | 6-n-Butyl-2-pyridyl | | |
| 1.157 | 6-tert.-Butyl-2-pyridyl | | |
| 1.158 | 6-n-Hexyl-2-pyridyl | | |
| 1.159 | 6-Phenyl-2-pyridyl | | |
| 1.160 | 6-Benzyl-2-pyridyl | | |
| 1.161 | 6-Trifluormethyl-2-pyridyl | | |
| 1.162 | 6-Methoxy-2-pyridyl | | |
| 1.163 | 6-Chloro-2-pyridyl | | |
| 1.164 | 3,6-Dimethyl-2-pyridyl | | |
| 1.165 | 4,6-Dimethyl-2-pyridyl | | |
| 1.166 | 5,6-Dimethyl-2-pyridyl | | |
| 1.167 | 4-Phenyl-6-methyl-2-pyridyl | | |
| 1.168 | 3,4-Dichloro-6-methyl-2-pyridyl | | |
| 1.169 | 3,4,5-Trichloro-6-methyl-2-pyridyl | | |
| 1.170 | 4-Trifluormethyl-6-methyl-2-pyridyl | | |
| 1.171 | 3-Acetyl-4,6-dimethyl-2-pyridyl | | |
| 1.172 | 3-Cyano-6-methyl-2-pyridyl | | |
| 1.173 | 3-Cyano-6-phenyl-2-pyridyl | | |
| 1.174 | 3-Methyloxycarbonyl-2-pyridyl | | |
| 1.175 | 3-Methyloxycarbonyl-6-iso-propyl-2-pyridyl | | |
| 1.176 | 5-Trifluormethyl-2-pyridyl | | |
| 1.177 | 2-Chinolyl | | |
| 1.178 | 3-Methyl-2-chinolyl | | |
| 1.179 | 4-Phenyl-2-chinolyl | | |
| 1.180 | 8-Chloro-2-chinolyl | | |
| 1.181 | 4-Methyl-8-methoxy-2-chinolyl | | |
| 1.182 | 4-Chinolyl | | |
| 1.183 | 2,6-Dimethyl-4-chinolyl | | |
| 1.184 | 8-Chinolyl | | |
| 1.185 | 5,7-Dichloro-8-chinolyl | | |
| 1.186 | 2-Pyrimidinyl | | |
| 1.187 | 4-Trifluormethyl-2-pyrimidinyl | | |
| 1.188 | 4,6-Dimethyl-5-chloro-2-pyrimidinyl | | |
| 1.189 | 3,5-Dimethyl-4-pyrimidinyl | | |
| 1.190 | 2-Pyrazinyl | | |

Tabelle 1 (Fortsetzung)

| Nr. | $A-X-(CH_2)_n$ | Fp. | IR: $\nu(cm^{-1})$ |
|---|---|---|---|
| 1.191 | 2-Thienyl | | |
| 1.192 | 3-Thienyl | | |
| 1.193 | 3-Methyl-2-chinoxalinyl | | |
| 1.194 | 3-Phenyl-5-isoxazolyl | | |
| 1.195 | 2-Benzoxazolyl | | |
| 1.196 | 2-Benzthiazolyl | | |
| 1.197 | 4-Chlor-2-benzthiazolyl | | |
| 1.198 | 5-Chlor-2-benzthiazolyl | | |
| 1.199 | 6-Chlor-2-benzthiazolyl | | |
| 1.200 | 6-Methyl-2-benzthiazolyl | | |
| 1.201 | 6-Methoxy-2-benzthiazolyl | | |

Tabelle 2

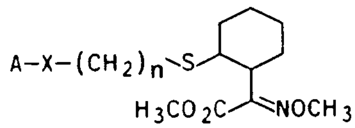

| Nr. | $A-X-(CH_2)_n$ | Fp. | IR: ν(cm$^{-1}$) |
|---|---|---|---|
| 2. 1 | H | | |
| 2. 2 | Methyl | | |
| 2. 3 | Ethyl | | |
| 2. 4 | Isopropyl | | |
| 2. 5 | Butyl | | |
| 2. 6 | sec.-Butyl | | |
| 2. 7 | t-Butyl | | |
| 2. 8 | Isobutyl | | |
| 2. 9 | Pentyl | | |
| 2.10 | Pivaloyl | | |
| 2.11 | Hexyl | | |
| 2.12 | Octyl | | |
| 2.13 | Decyl | | |
| 2.14 | Phenyl | Öl | 2935, 1738, 1722, 1152, 1043 |
| 2.15 | Benzyl | Öl | 2935, 1726, 1151, 1042 |
| 2.16 | Phenylethyl | | |
| 2.17 | Phenylpropyl | | |
| 2.18 | Phenylpentyl | | |
| 2.19 | 1-Naphthyl | | |
| 2.20 | 2-Naphthyl | | |
| 2.21 | 2-Bromphenyl | | |
| 2.22 | 3-Bromphenyl | | |
| 2.23 | 4-Bromphenyl | | |
| 2.24 | 2-Chlorphenyl | | |
| 2.25 | 3-Chlorphenyl | | |
| 2.26 | 4-Chlorphenyl | | |
| 2.27 | 2-Fluorphenyl | | |
| 2.28 | 3-Fluorphenyl | | |
| 2.29 | 4-Fluorphenyl | | |
| 2.30 | 2-Methylphenyl | | |
| 2.31 | 3-Methylphenyl | | |
| 2.32 | 4-Methylphenyl | | |
| 2.33 | 2-Ethyl-phenyl | | |
| 2.34 | 3-Ethyl-phenyl | | |
| 2.35 | 4-Ethyl-phenyl | | |

Tabelle 2 (Fortsetzung)

| Nr. | A-X-$(CH_2)_n$ | Fp. | IR: $\nu(cm^{-1})$ |
|---|---|---|---|
| 2.36 | 2-iso-Propyl-phenyl | | |
| 2.37 | 3-iso-Propyl-phenyl | | |
| 2.38 | 4-iso-Propyl-phenyl | | |
| 2.39 | 2-tert.-Butyl-phenyl | | |
| 2.40 | 3-tert.-Butyl-phenyl | | |
| 2.41 | 4-tert.-Butyl-phenyl | | |
| 2.42 | 4-Butyl-phenyl | | |
| 2.43 | 4-Hexyl-phenyl | | |
| 2.44 | 4-Nonyl-phenyl | | |
| 2.45 | 4-Decyl-phenyl | | |
| 2.46 | 2-Methoxy-phenyl | | |
| 2.47 | 3-Methoxy-phenyl | | |
| 2.48 | 4-Methoxy-phenyl | | |
| 2.49 | 2-Trifluormethyl-phenyl | | |
| 2.50 | 3-Trifluormethyl-phenyl | | |
| 2.51 | 4-Trifluormethyl-phenyl | | |
| 2.52 | 2-Formylphenyl | | |
| 2.53 | 3-Formylphenyl | | |
| 2.54 | 4-Formylphenyl | | |
| 2.55 | 2-Allyl-O-N=CH-phenyl | | |
| 2.56 | 3-Allyl-O-N=CH-phenyl | | |
| 2.57 | 4-Allyl-O-N-CH-phenyl | | |
| 2.58 | 2-Nitro-phenyl | | |
| 2.59 | 3-Nitro-phenyl | | |
| 2.60 | 2,4-Dichlorphenyl | | |
| 2.61 | 2,5-Dichlor-phenyl | | |
| 2.62 | 2,6-Dichlor-phenyl | | |
| 2.63 | 3,4-Dichlor-phenyl | | |
| 2.64 | 2,3-Dichlor-phenyl | | |
| 2.65 | 3,5-Dichlor-phenyl | | |
| 2.66 | 2,3,4-Trichlor-phenyl | | |
| 2.67 | 2,4,5-Trichlor-phenyl | | |
| 2.68 | 2,4,6-Trichlor-phenyl | | |
| 2. 69 | 2,3,4,6-Tetrachlor-phenyl | | |
| 2. 70 | 2,3,4,5,6-Pentachlor-phenyl | | |
| 2. 71 | 2,3,4,5-Tetrafluor-phenyl | | |
| 2. 72 | 2,3,5,6-Tetrafluor-phenyl | | |
| 2. 73 | 2,3,4,5,6-Pentafluor-phenyl | | |
| 2. 74 | 2-Chlor, 4-Fluor-phenyl | | |

Tabelle 2 (Fortsetzung)

| Nr. | A-X-$(CH_2)_n$ | Fp. | IR: $\nu(cm^{-1})$ |
|---|---|---|---|
| 2. 75 | 3-Chlor, 4-Fluor-phenyl | | |
| 2. 76 | 2-Chlor, 6-Methyl-phenyl | | |
| 2. 77 | 4-Chlor, 2-Methyl-phenyl | | |
| 2. 78 | 2,4-Dichlor, 5-Methyl-phenyl | | |
| 2. 79 | 4-Chlor, 2,5-Dimethyl-phenyl | | |
| 2. 80 | 4-Brom, 2-Methyl-phenyl | | |
| 2. 81 | 3,5-Bistrifluormethyl-phenyl | | |
| 2. 82 | 2,3-Dimethyl-phenyl | | |
| 2. 83 | 2,4-Dimethyl-phenyl | | |
| 2. 84 | 2,5-Dimethyl-phenyl | | |
| 2. 85 | 2,6-Dimethyl-phenyl | | |
| 2. 86 | 3,4-Dimethyl-phenyl | | |
| 2. 87 | 3,5-Dimethyl-phenyl | | |
| 2. 88 | 2,4,5-Trimethyl-phenyl | | |
| 2. 89 | 2,6-Diethyl-phenyl | | |
| 2. 90 | 2,4-Di-tert.-Butyl-phenyl | | |
| 2. 91 | 2,5-Dimethoxy-phenyl | | |
| 2. 92 | 3,4-Dimethoxy-phenyl | | |
| 2. 93 | 2-Methyl, 4-tert.-Butyl-phenyl | | |
| 2. 94 | 2-Methoxycarbonyl-phenyl | | |
| 2. 95 | 2-Ethoxycarbonyl-phenyl | | |
| 2. 96 | 2-Propoxycarbonyl-phenyl | | |
| 2. 97 | 2-Butoxycarbonyl-phenyl | | |
| 2. 98 | 2-Cyano-phenyl | | |
| 2. 99 | 3-Cyano-phenyl | | |
| 2.100 | 4-Cyano-phenyl | | |
| 2.101 | 1-Naphthylmethyl | | |
| 2.102 | 2-Naphthylmethyl | | |
| 2.103 | 2-Brombenzyl | | |
| 2.104 | 3-Brombenzyl | | |
| 2.105 | 4-Brombenzyl | | |
| 2.106 | 2-Chlorbenzyl | | |
| 2.107 | 3-Chlorbenzyl | | |
| 2.108 | 4-Chlorbenzyl | | |
| 2.109 | 2-Fluorbenzyl | | |
| 2.110 | 3-Fluorbenzyl | | |
| 2.111 | 4-Fluorbenzyl | | |
| 2.112 | 2-Methylbenzyl | | |
| 2.113 | 3-Methylbenzyl | | |

Tabelle 2 (Fortsetzung)

| Nr. | $A-X-(CH_2)_n$ | Fp. | IR: $\nu(cm^{-1})$ |
|---|---|---|---|
| 2.114 | 4-Methylbenzyl | | |
| 2.115 | 2-Methoxybenyzl | | |
| 2.116 | 3-Methoxybenzyl | | |
| 2.117 | 4-Methoxybenzyl | | |
| 2.118 | 4-Ethoxybenzyl | | |
| 2.119 | 4-Butyloxybenzyl | | |
| 2.120 | 4-Benzyloxybenzyl | | |
| 2.121 | 2-Trifluormethyl-benzyl | | |
| 2.122 | 3-Trifluormethyl-benzyl | | |
| 2.123 | 4-Trifluormethyl-benzyl | | |
| 2.124 | 2-Formylbenzyl | | |
| 2.125 | 3-Formylbenzyl | | |
| 2.126 | 4-Formylbenzyl | | |
| 2.127 | 2-Allyl-O-N=CH-benzyl | | |
| 2.128 | 3-Allyl-O-N=CH-benzyl | | |
| 2.129 | 4-Allyl-O-N=CH-benzyl | | |
| 2.130 | 2-Nitrobenzyl | | |
| 2.131 | 3-Nitrobenzyl | | |
| 2.132 | 2,4-Dichlorbenzyl | | |
| 2.133 | 2,6-Dichlorbenzyl | | |
| 2.134 | 3,4-Dichlorbenzyl | | |
| 2.135 | 2,3-Dichlorbenzyl | | |
| 2.136 | 3,5-Dichlorbenzyl | | |
| 2.137 | 2,3-Dimethylbenzyl | | |
| 2.138 | 2,4-Dimethylbenzyl | | |
| 2.139 | 2,5-Dimethylbenzyl | | |
| 2.140 | 2,6-Dimethylbenzyl | | |
| 2.141 | 3,4-Dimethylbenzyl | | |
| 2.142 | 3,5-Dimethylbenzyl | | |
| 2.143 | 4-Chlor-2-methylbenzyl | | |
| 2.144 | 2-Methoxycarbonyl-benzyl | | |
| 2.145 | 2-Ethoxycarbonyl-benzyl | | |
| 2.146 | 2-Butoxycarbonyl-benzyl | | |
| 2.147 | 2-Cyano-benzyl | | |
| 2.148 | 3-Cyano-benzyl | | |
| 2.149 | 4-Cyano-benzyl | | |
| 2.150 | 2-Pyridyl | | |
| 2.151 | 3-Pyridyl | | |
| 2.152 | 4-Pyridyl | | |

Tabelle 2 (Fortsetzung)

| Nr. | $A-X-(CH_2)_n$ | Fp. | IR: $\nu(cm^{-1})$ |
|---|---|---|---|
| 2.153 | 6-Methyl-2-pyridyl | | |
| 2.154 | 6-Ethyl-2-pyridyl | | |
| 2.155 | 6-n-Propyl-2-pyridyl | | |
| 2.156 | 6-n-Butyl-2-pyridyl | | |
| 2.157 | 6-tert.-Butyl-2-pyridyl | | |
| 2.158 | 6-n-Hexyl-2-pyridyl | | |
| 2.159 | 6-Phenyl-2-pyridyl | | |
| 2.160 | 6-Benzyl-2-pyridyl | | |
| 2.161 | 6-Trifluormethyl-2-pyridyl | | |
| 2.162 | 6-Methoxy-2-pyridyl | | |
| 2.163 | 6-Chloro-2-pyridyl | | |
| 2.164 | 3,6-Dimethyl-2-pyridyl | | |
| 2.165 | 4,6-Dimethyl-2-pyridyl | | |
| 2.166 | 5,6-Dimethyl-2-pyridyl | | |
| 2.167 | 4-Phenyl-6-methyl-2-pyridyl | | |
| 2.168 | 3,4-Dichloro-6-methyl-2-pyridyl | | |
| 2.169 | 3,4,5-Trichloro-6-methyl-2-pyridyl | | |
| 2.170 | 4-Trifluormethyl-6-methyl-2-pyridyl | | |
| 2.171 | 3-Acetyl-4,6-dimethyl-2-pyridyl | | |
| 2.172 | 3-Cyano-6-methyl-2-pyridyl | | |
| 2.173 | 3-Cyano-6-phenyl-2-pyridyl | | |
| 2.174 | 3-Methyloxycarbonyl-2-pyridyl | | |
| 2.175 | 3-Methyloxycarbonyl-6-iso-propyl-2-pyridyl | | |
| 2.176 | 5-Trifluormethyl-2-pyridyl | | |
| 2.177 | 2-Chinolyl | | |
| 2.178 | 3-Methyl-2-chinolyl | | |
| 2.179 | 4-Phenyl-2-chinolyl | | |
| 2.180 | 8-Chloro-2-chinolyl | | |
| 2.181 | 4-Methyl-8-methoxy-2-chinolyl | | |
| 2.182 | 4-Chinolyl | | |
| 2.183 | 2,6-Dimethyl-4-chinolyl | | |
| 2.184 | 8-Chinolyl | | |
| 2.185 | 5,7-Dichloro-8-chinolyl | | |
| 2.186 | 2-Pyrimidinyl | | |
| 2.187 | 4-Trifluormethyl-2-pyrimidinyl | | |
| 2.188 | 4,6-Dimethyl-5-chloro-2-pyrimidinyl | | |
| 2.189 | 3,5-Dimethyl-4-pyrimidinyl | | |
| 2.190 | 2-Pyrazinyl | | |
| 2.191 | 2-Thienyl | | |

Tabelle 2 (Fortsetzung)

| Nr. | $A-X-(CH_2)_n$ | Fp. | IR: $\nu(cm^{-1})$ |
|---|---|---|---|
| 2.192 | 3-Thienyl | | |
| 2.193 | 3-Methyl-2-chinoxalinyl | | |
| 2.194 | 3-Phenyl-5-isoxazolyl | | |
| 2.195 | 2-Benzoxazolyl | | |
| 2.196 | 2-Benzthiazolyl | | |
| 2.197 | 4-Chlor-2-benzthiazolyl | | |
| 2.198 | 5-Chlor-2-benzthiazolyl | | |
| 2.199 | 6-Chlor-2-benzthiazolyl | | |
| 2.200 | 6-Methyl-2-benzthiazolyl | | |
| 2.201 | 6-Methoxy-2-benzthiazolyl | | |

Tabelle 3

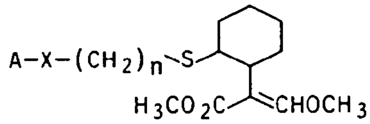

| Nr. | A-X-$(CH_2)_n$ | Fp. | IR: ν($cm^{-1}$) |
|---|---|---|---|
| 3. 1 | n-Butyl | | |
| 3. 2 | Phenyl | | |
| 3. 3 | 2-Methoxyphenyl | | |
| 3. 4 | 3-Methoxyphenyl | | |
| 3. 5 | 4-Methoxyphenyl | | |
| 3. 6 | 2-Chlorphenyl | | |
| 3. 7 | 3-Chlorphenyl | | |
| 3. 8 | 4-Chlorphenyl | | |
| 3. 9 | 2-Methylphenyl | | |
| 3.10 | 3-Methylphenyl | | |
| 3.11 | 4-Methylphenyl | | |
| 3.12 | 2,3-Dimethylphenyl | | |
| 3.13 | 2,4-Dimethylphenyl | | |
| 3.14 | 2,5-Dimethylphenyl | | |
| 3.15 | 2,6-Dimethylphenyl | | |
| 3.16 | 3,4-Dimethylphenyl | | |
| 3.17 | 3,5-Dimethylphenyl | | |
| 3.18 | 4-Chlor-2-methylphenyl | | |
| 3.19 | 4-Methoxycarbonylphenyl | | |
| 3.20 | 4-Ethoxycarbonylphenyl | | |
| 3.21 | 4-Butoxycarbonylphenyl | | |
| 3.22 | 2-Allyl-O-N=CH-phenyl | | |
| 3.23 | 3-Allyl-O-N=CH-phenyl | | |
| 3.24 | 4-Allyl-O-N=CH-phenyl | | |
| 3.25 | 2-Pyridyl | | |
| 3.26 | 6-Methyl-2-pyridyl | | |
| 3.27 | 6-Chlor-2-pyridyl | | |
| 3.28 | 4-Chlor-2-benzthiazolyl | | |
| 3.29 | 5-Chlor-2-benzthiazolyl | | |
| 3.30 | 6-Chlor-2-benzthiazolyl | | |
| 3.31 | 2-Methoxybenzyl | | |
| 3.32 | 3-Methoxybenzyl | | |
| 3.33 | 4-Methoxybenzyl | | |
| 3.34 | 2-Chlorbenzol | | |
| 3.35 | 3-Chlorbenzol | | |

Tabelle 3 (Fortsetzung)

| Nr. | $A-X-(CH_2)_n$ | Fp. | IR: $\nu(cm^{-1})$ |
|---|---|---|---|
| 3.36 | 4-Chlorbenzyl | | |
| 3.37 | 2-Methylbenzyl | | |
| 3.38 | 3-Methylbenzyl | | |
| 3.39 | 4-Methylbenzyl | | |
| 3.40 | 2,3-Dimethylbenzyl | | |
| 3.41 | 2,4-Dimethylbenzyl | | |
| 3.42 | 2,5-Dimethylbenzyl | | |
| 3.43 | 2,6-Dimethylbenzyl | | |
| 3.44 | 3,4-Dimethylbenzyl | | |
| 3.45 | 3,5-Dimethylbenzyl | | |
| 3.46 | 4-Chlor-2-methylbenzyl | | |
| 3.47 | 4-Methoxycarbonylbenzyl | | |
| 3.48 | 4-Ethoxycarbonylbenzyl | | |
| 3.49 | 4-Butoxycarbonylbenzyl | | |
| 3.50 | 2-Allyl-O-N=CH-phenyl | | |
| 3.51 | 3-Allyl-O-N=CH-phenyl | | |
| 3.52 | 4-Allyl-O-N=CH-phenyl | | |

Tabelle 4

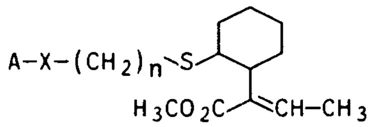

| Nr. | $A-X-(CH_2)_n$ | Fp. | IR: ν($cm^{-1}$) |
|---|---|---|---|
| 4. 1 | n-Butyl | | |
| 4. 2 | Phenyl | | |
| 4. 3 | 2-Methoxyphenyl | | |
| 4. 4 | 3-Methoxyphenyl | | |
| 4. 5 | 4-Methoxyphenyl | | |
| 4. 6 | 2-Chlorphenyl | | |
| 4. 7 | 3-Chlorphenyl | | |
| 4. 8 | 4-Chlorphenyl | | |
| 4. 9 | 2-Methylphenyl | | |
| 4.10 | 3-Methylphenyl | | |
| 4.11 | 4-Methylphenyl | | |
| 4.12 | 2,3-Dimethylphenyl | | |
| 4.13 | 2,4-Dimethylphenyl | | |
| 4.14 | 2,5-Dimethylphenyl | | |
| 4.15 | 2,6-Dimethylphenyl | | |
| 4.16 | 3,4-Dimethylphenyl | | |
| 4.17 | 3,5-Dimethylphenyl | | |
| 4.18 | 4-Chlor-2-methylphenyl | | |
| 4.19 | 4-Methoxycarbonylphenyl | | |
| 4.20 | 4-Ethoxycarbonylphenyl | | |
| 4.21 | 4-Butoxycarbonylphenyl | | |
| 4.22 | 2-Allyl-O-N=CH-phenyl | | |
| 4.23 | 3-Allyl-O-N=CH-phenyl | | |
| 4.24 | 4-Allyl-O-N=CH-phenyl | | |
| 4.25 | 2-Pyridyl | | |
| 4.26 | 6-Methyl-2-pyridyl | | |
| 4.27 | 6-Chlor-2-pyridyl | | |
| 4.28 | 4-Chlor-2-benzthiazolyl | | |
| 4.29 | 5-Chlor-2-benzthiazolyl | | |
| 4.30 | 6-Chlor-2-benzthiazolyl | | |
| 4.31 | 2-Methoxybenzyl | | |
| 4.32 | 3-Methoxybenzyl | | |
| 4.33 | 4-Methoxybenzyl | | |
| 4.34 | 2-Chlorbenzol | | |
| 4.35 | 3-Chlorbenzol | | |

Tabelle 4 (Fortsetzung)

| Nr. | A-X-$(CH_2)_n$ | Fp. | IR: $\nu(cm^{-1})$ |
|---|---|---|---|
| 4.36 | 4-Chlorbenzyl | | |
| 4.37 | 2-Methylbenzyl | | |
| 4.38 | 3-Methylbenzyl | | |
| 4.39 | 4-Methylbenzyl | | |
| 4.40 | 2,3-Dimethylbenzyl | | |
| 4.41 | 2,4-Dimethylbenzyl | | |
| 4.42 | 2,5-Dimethylbenzyl | | |
| 4.43 | 2,6-Dimethylbenzyl | | |
| 4.44 | 3,4-Dimethylbenzyl | | |
| 4.45 | 3,5-Dimethylbenzyl | | |
| 4.46 | 4-Chlor-2-methylbenzyl | | |
| 4.47 | 4-Methoxycarbonylbenzyl | | |
| 4.48 | 4-Ethoxycarbonylbenzyl | | |
| 4.49 | 4-Butoxycarbonylbenzyl | | |
| 4.50 | 2-Allyl-O-N=CH-phenyl | | |
| 4.51 | 3-Allyl-O-N=CH-phenyl | | |
| 4.52 | 4-Allyl-O-N=CH-phenyl | | |

Tabelle 5

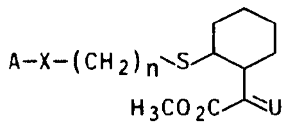

| Nr. | A-X-$(CH_2)_n$ | U | Fp. | IR: ν/cm$^{-1}$) |
|---|---|---|---|---|
| 5. 1 | 2-Methylphenyl | $CH_2$ | | |
| 5. 2 | 3-Methoxyphenyl | $CH_2$ | | |
| 5. 3 | 4-Chlorphenyl | $CH_2$ | | |
| 5. 4 | 2-Allyl-O-N=CH-phenyl | $CH_2$ | | |
| 5. 5 | 3-Methoxycarbonylphenyl | $CH_2$ | | |
| 5. 6 | 6-Chlor-2-pyridyl | $CH_2$ | | |
| 5. 7 | 6-Chlor-2-benzthiazolyl | $CH_2$ | | |
| 5. 8 | 2-Methylbenzyl | $CH_2$ | | |
| 5. 9 | 3-Methoxybenzyl | $CH_2$ | | |
| 5.10 | 4-Chlorbenzyl | $CH_2$ | | |
| 5.11 | 2-Allyl-O-N=CH-benzyl | $CH_2$ | | |
| 5.12 | 3-Methoxycarbonyl-benzyl | $CH_2$ | | |
| 5.13 | 2-Methylphenyl | $CH-CH_2-CH_3$ | | |
| 5.14 | 3-Methoxyphenyl | $CH-CH_2-CH_3$ | | |
| 5.15 | 4-Chlorphenyl | $CH-CH_2-CH_3$ | | |
| 5.16 | 2-Allyl-O-N=CH-phenyl | $CH-CH_2-CH_3$ | | |
| 5.17 | 3-Methoxycarbonylphenyl | $CH-CH_2-CH_3$ | | |
| 5.18 | 6-Chlor-2-pyridyl | $CH-CH_2-CH_3$ | | |
| 5.19 | 6-Chlor-2-benzthiazolyl | $CH-CH_2-CH_3$ | | |
| 5.20 | 2-Methylbenzyl | $CH-CH_2-CH_3$ | | |
| 5.21 | 3-Methoxybenzyl | $CH-CH_2-CH_3$ | | |
| 5.22 | 4-Chlorbenzyl | $CH-CH_2-CH_3$ | | |
| 5.23 | 2-Allyl-O-N=CH-benzyl | $CH-CH_2-CH_3$ | | |
| 5.24 | 3-Methoxycarbonyl-benzyl | $CH-CH_2-CH_3$ | | |
| 5.25 | 2-Methylphenyl | $N-NH-CH_3$ | | |
| 5.26 | 3-Methoxyphenyl | $N-NH-CH_3$ | | |
| 5.27 | 4-Chlorphenyl | $N-NH-CH_3$ | | |
| 5.28 | 2-Allyl-O-N=CH-phenyl | $N-NH-CH_3$ | | |
| 5.29 | 3-Methoxycarbonylphenyl | $N-NH-CH_3$ | | |
| 5.30 | 6-Chlor-2-pyridyl | $N-NH-CH_3$ | | |
| 5.31 | 6-Chlor-2-benzthiazolyl | $N-NH-CH_3$ | | |
| 5.32 | 2-Methylbenzyl | $N-NH-CH_3$ | | |
| 5.33 | 3-Methoxybenzyl | $N-NH-CH_3$ | | |
| 5.34 | 4-Chlorbenzyl | $N-NH-CH_3$ | | |
| 5.35 | 2-Allyl-O-N=CH-benzyl | $N-NH-CH_3$ | | |

Tabelle 5 (Fortsetzung)

| Nr. | $A-X-(CH_2)_n$ | U | Fp. | IR: v/cm$^{-1}$) |
|---|---|---|---|---|
| 5.36 | 3-Methoxycarbonyl-benzyl | $N-NH-CH_3$ | | |
| 5.37 | 2-Methylphenyl | $CH-S-CH_3$ | | |
| 5.38 | 3-Methoxyphenyl | $CH-S-CH_3$ | | |
| 5.39 | 4-Chlorphenyl | $CH-S-CH_3$ | | |
| 5.40 | 2-Allyl-O-N=CH-phenyl | $CH-S-CH_3$ | | |
| 5.41 | 3-Methoxycarbonylphenyl | $CH-S-CH_3$ | | |
| 5.42 | 6-Chlor-2-pyridyl | $CH-S-CH_3$ | | |
| 5.43 | 6-Chlor-2-benzthiazolyl | $CH-S-CH_3$ | | |
| 5.44 | 2-Methylbenzyl | $CH-S-CH_3$ | | |
| 5.45 | 3-Methoxybenzyl | $CH-S-CH_3$ | | |
| 5.46 | 4-Chlorbenzyl | $CH-S-CH_3$ | | |
| 5.47 | 2-Allyl-O-N=CH-benzyl | $CH-S-CH_3$ | | |
| 5.48 | 3-Methoxycarbonyl-benzyl | $CH-S-CH_3$ | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzubefall zu schützenden Pflanzen, Saatgüter, Materialien oder der Erdboden mit einer fungizid wirksamen Menge des Wirkstoffs behandelt. Es werden die Insekten oder ihr Lebensraum mit einer insektizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Sub-

stanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden und insektiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 20 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2.15 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2.15 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 2.15 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 2.15 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 2.15 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 2.15 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2.15 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 2.15 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2.15 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß der Wirkstoff 2.15 bei der Anwendung als 0,025 %ige (Gew.%) Spritzbrühe eine gute fungizide Wirkung zeigt (100 %).

## Patentansprüche

1. Ungesättigte Cyclohexylessigsäurederivate der Formel I

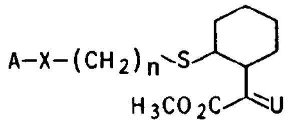

I

in der
U =$NOCH_3$, =$CHOCH_3$, =$CH_2$, =$CH$-$CH_3$, =$CH$-$CH_2$-$CH_3$, =N-NH-$CH_3$ oder =CH-S-$CH_3$ bedeutet,
n die Zahlen 0 bis 10 bedeutet,
A Wasserstoff oder die gegebenenfalls substituierten Reste Alkyl, Cycloalkyl, Aryl oder Hetaryl bedeutet und
X im Fall n=O eine Einfachbindung und im Fall n verschieden von 0 Sauerstoff oder Schwefel oder eine Einfachbindung bedeutet, sowie deren pflanzenverträgliche Säureadditionsprodukte und Basenadditionsprodukte.

2. Verbindungen der Formel II

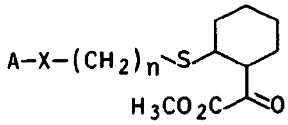

II

in der
n die Zahlen 0 bis 10 bedeutet,
A Wasserstoff oder die gegebenenfalls substituierten Reste Alkyl, Cycloalkyl, Aryl oder Hetaryl bedeutet und
X im Fall n=O eine Einfachbindung und im Fall n verschieden von 0 Sauerstoff oder Schwefel oder eine Einfachbindung ist.

3. Verfahren zur Herstellung eines Cyclohexylessigesters der Formel I

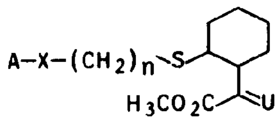

I

in der
U $=NOCH_3$, $=CH\text{-}OCH_3$, $=CH_2$, $=CH\text{-}CH_3$, $=CH\text{-}CH_2\text{-}CH_3$ oder $=N\text{-}NH\text{-}CH_3$ bedeutet,
n die Zahlen 0 bis 10 beddeutet
A Wasserstoff oder die gegebenenfalls substituierten Reste Alkyl, Cycloalkyl, Aryl oder Hetaryl bedeutet und
X
im Fall n=O eine Einfachbindung und im Fall n verschieden von 0 Sauerstoff oder Schwefel oder eine Einfachbindung bedeutet,
dadurch gekennzeichnet, daß man eine Verbindung der Formel II

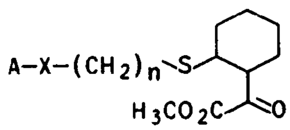
II

in der n, A und X die oben angegebenen Bedeutungen haben, mit $CH_3\text{-}O\text{-}NH_3Cl$, $H_2N\text{-}NH\text{-}CH_3$ oder mit $(C_6H_5)_3$ PT umsetzt, wobei T $-CH_3Hal$, $-CH_2\text{-}CH_3Hal$, $-CH_2\text{-}CH_2\text{-}CH_3Hal$, $-CH_2\text{-}O\text{-}CH_3Hal$ und Hal Halogen (Cl, Br) bedeutet.

**4.** Verbindung der Formel II gemäß Anspruch 2, in der
n = 1,
A Phenyl und
X eine Einfachbindung bedeutet.

**5.** Verbindung der Formel I gemäß Anspruch 1, in der
U $=NOCH_3$ bedeutet
n = 1,
A Phenyl und
X eine Einfachbindung bedeutet.

**6.** Fungizid, dadurch gekennzeichnet, daß es einen inerten Trägerstoff und eine fungizid wirksame Menge einer Verbindung der Formel I

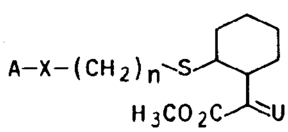
I

enthält, in der
U $=NOCH_3$, $=CHOCH_3$, $=CH_2$, $=CH\text{-}CH_3$, $=CH\text{-}CH_2\text{-}CH_3$, $=N\text{-}NH\text{-}CH_3$ oder $=CH\text{-}S\text{-}CH_3$ bedeutet,
n die Zahlen 0 bis 10 bedeutet,
A Wasserstoff oder die gegebenenfalls substituierten Reste Alkyl, Cycloalkyl, Aryl oder Hetaryl bedeutet und
X im Fall n=O eine Einfachbindung und im Fall n verschieden von 0 Sauerstoff oder Schwefel oder eine Einfachbindung bedeutet, oder deren pflanzenverträgliches Säureadditionsprodukt oder Basenadditionsprodukt.

**7.** Insektizid, dadurch gekennzeichnet, daß es einen inerten Trägerstoff und eine insektizid wirksame Menge einer Verbindung der Formel I

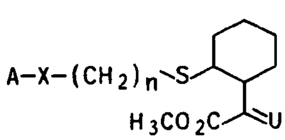
I

enthält, in der
in der
U $=NOCH_3$, $=CHOCH_3$, $=CH_2$, $=CH-CH_3$, $=CH-CH_2-CH_3$, $=N-NH-CH_3$ oder $=CH-S-CH_3$ bedeutet,
n die Zahlen 0 bis 10 bedeutet,
A Wasserstoff oder die gegebenenfalls substituierten Reste Alkyl, Cycloalkyl, Aryl oder Hetaryl bedeutet und
X im Fall n=O eine Einfachbindung und im Fall n verschieden von 0 Sauerstoff oder Schwefel oder eine Einfachbindung bedeutet, oder deren pflanzenverträgliches Säureadditionsprodukt oder Basenadditionsprodukt.

8. Verfahren zur Bekämpfung von Pilzen, dadurch gekennzeichnet, daß man die Pilze oder die vor Pilzbefall zu schützenden Pflanzen, Saatgüter, Materialien oder den Erdboden behandelt mit einer fungizid wirksamen Menge einer Verbindung der Formel I

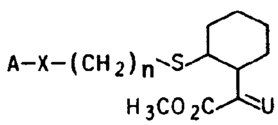

I

in der
U $=NOCH_3$, $=CHOCH_3$, $=CH_2$, $=CH-CH_3$, $=CH-CH_2-CH_3$, $=N-NH-CH_3$ oder $=CH-S-CH_3$ bedeutet,
n die Zahlen 0 bis 10 bedeutet,
A Wasserstoff oder die gegebenenfalls substituierten Reste Alkyl, Cycloalkyl, Aryl oder Hetaryl bedeutet und
X im Fall n=O eine Einfachbindung und im Fall n verschieden von 0 Sauerstoff oder Schwefel oder eine Einfachbindung bedeutet, oder deren pflanzenverträglichem Säureadditionsprodukt oder Basenadditionsprodukt.

## Claims

1. An unsaturated cyclohexylacetic acid derivative of the formula I

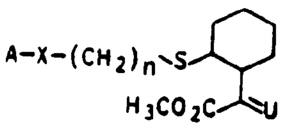

I

where
U is $=NOCH_3$, $=CHOCH_3$, $=CH_2$, $=CH-CH_3$, $=CH-CH_2-CH_3$, $=N-NH-CH_3$ or $=CH-S-CH_3$,
n is from 0 to 10,
A is hydrogen or substituted or unsubstituted alkyl, cycloalkyl, aryl or hetaryl, and
X is a single bond when n is 0, and oxygen, sulfur or a single bond when n is not 0, or a plant-compatible acid addition product or base addition product thereof.

2. A compound of the formula II

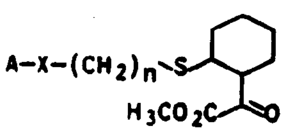

II

where
n is from 0 to 10,
A is hydrogen or substituted or unsubstituted alkyl, cycloalkyl, aryl or hetaryl, and

X is a single bond when n is 0, and oxygen, sulfur or a single bond when n is not 0.

3. A process for preparing a cyclohexylacetic ester of the formula I

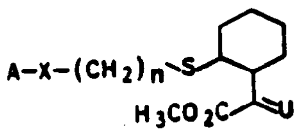

I

where
U is $=NOCH_3$, $=CH-OCH_3$, $=CH_2$, $=CH-CH_3$, $=CH-CH_2-CH_3$ or $=N-NH-CH_3$,
n is from 0 to 10,
A is hydrogen or substituted or unsubstituted alkyl, cycloalkyl, aryl or hetaryl, and
X is a single bond when n is 0, and oxygen, sulfur or a single bond when n is not 0,
which comprises reacting a compound of the formula II

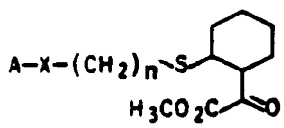

II

where n, A and X are as defined above, with $CH_3-O-NH_3Cl$ or $H_2N-NH-CH_3$ or with $(C_6H_5)_3$ PT where T is $-CH_3hal$, $-CH_2-CH_3hal$, $-CH_2-CH_2-CH_3hal$ or $-CH_2-O-CH_3hal$, and hal is halogen (Cl, Br).

4. A compound of the formula II as claimed in claim 2, where
n is 1,
A is phenyl, and
X is a single bond.

5. A compound of the formula I as claimed in claim 1, where
U is $=NOCH_3$,
n is 1,
A is phenyl, and
X is a single bond.

6. A fungicide containing an inert carrier and a fungicidally effective amount of a compound of the formula I

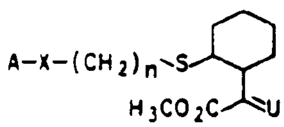

I

where
U is $=NOCH_3$, $=CHOCH_3$, $=CH_2$, $=CH-CH_3$, $=CH-CH_2-CH_3$, $=N-NH-CH_3$ or $=CH-S-CH_3$,
n is from 0 to 10,
A is hydrogen or substituted or unsubstituted alkyl, cycloalkyl, aryl or hetaryl, and
X is a single bond when n is 0, and oxygen, sulfur or a single bond when n is not 0, or a plant-compatible acid addition product or base addition product thereof.

7. An insecticide containing an inert carrier and an insecticidally effective amount of a compound of the formula I

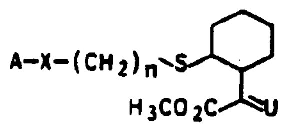

I

where
U is $=NOCH_3$, $=CHOCH_3$, $=CH_2$, $=CH\text{-}CH_3$, $=CH\text{-}CH_2\text{-}CH_3$, $=N\text{-}NH\text{-}CH_3$ or $=CH\text{-}S\text{-}CH_3$,
n is from 0 to 10,
A is hydrogen or substituted or unsubstituted alkyl, cycloalkyl, aryl or hetaryl, and
X is a single bond when n is 0, and oxygen, sulfur or a single bond when n is not 0, or a plant-compatible acid addition product or base addition product thereof.

**8.** A method for controlling fungi, wherein the fungi, or the plants, seed, materials or soil to be protected against fungal attack, are treated with a fungicidally effective amount of a compound of the formula I

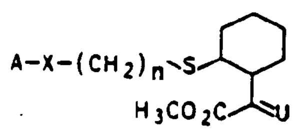

I

where
U is $=NOCH_3$, $=CHOCH_3$, $=CH_2$, $=CH\text{-}CH_3$, $=CH\text{-}CH_2\text{-}CH_3$, $=N\text{-}NH\text{-}CH_3$ or $=CH\text{-}S\text{-}CH_3$,
n is from 0 to 10,
A is hydrogen or substituted or unsubstituted alkyl, cycloalkyl, aryl or hetaryl, and
X is a single bond when n is 0, and oxygen, sulfur or a single bond when n is not 0, or a plant-compatible acid addition product or base addition product thereof.

## Revendications

**1.** Dérivés d'acide cyclohexylacétique insaturées de formule I

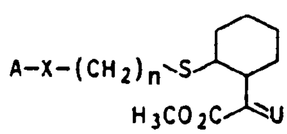

I

dans laquelle
U = $NOCH_3$, $=CHOCH_3$, $=CH_2$, $=CH\text{-}CH_3$, $=CH\text{-}CH_2\text{-}CH_3$, = $N\text{-}NH\text{-}CH_3$ ou = $CH\text{-}S\text{-}CH_3$
n représente un nombre de 0 à 10,
A représente hydrogène ou les restes alkyle, cycloalkyle, aryle ou hétaryle éventuellement substitués,
X, au cas où n = 0, est mis pour une liaison simple et au cas où n est différent de 0, est mis pour oxygène ou soufre ou une liaison simple, ainsi que leurs produits d'addition d'acide et produits d'addition de base tolérés par les plantes.

**2.** Composés de formule II

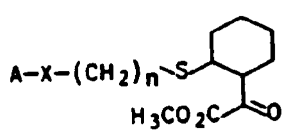

· II

dans laquelle
n représente un nombre de 0 à 10,

A représente hydrogène ou les restes alkyle, cycloalkyle, aryle ou hétaryle éventuellement substitués, et X, au cas où n = 0, est mis pour une liaison simple et, au cas où n est différent de 0, pour oxygène ou soufre ou une liaison simple.

**3.** Procédé de préparation d'un ester cyclohexylacétique de formule I

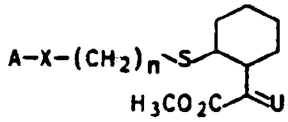

I

dans laquelle
U = $NOCH_3$, = $CHOCH_3$, =$CH_2$, = CH-$CH_3$, = CH-$CH_2$-$CH_3$, = N-NH-$CH_3$ ou = CH-S-$CH_3$,
n représente un nombre de 0 à 10,
A représente hydrogène ou les restes alkyle, cycloalkyle, aryle ou hétaryle éventuellement substitués,
X, au cas où n = 0, est mis pour une liaison simple et, au cas où n est différent de 0, est mis pour oxygène ou soufre ou une liaison simple, caractérisé par le fait que l'on fait réagir un composé de formule II

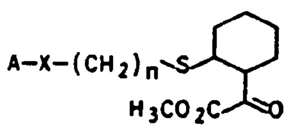

II

dans laquelle n, A et X ont les significations indiquées plus haut, avec $CH_3$-O-$NH_3Cl$, $H_2N$-NH-$CH_3$ ou avec $(C_6H_5)_3PT$, T représentant -$CH_3Hal$, -$CH_2$-$CH_3Hal$, -$CH_2$-$CH_2$-$CH_3Hal$, -$CH_2$-O-$CH_3Hal$ et Hal étant mis pour halogène (Cl, Br).

**4.** Composé de formule II, selon la revendication 2, dans laquelle
n = 1
A = phényle et
X = liaison simple.

**5.** Composé de formule I, selon la revendication 1, dans laquelle
U = $NOCH_3$
n = 1
A = phényle et
X = liaison simple.

**6.** Fongicide caractérisé par le fait qu'il contient un véhicule inerte et une quantité efficace en tant que fongicide d'un composé de formule I

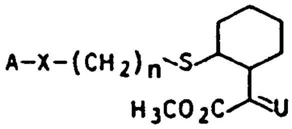

I

dans laquelle
U = $NOCH_3$, =$CHOCH_3$, =$CH_2$, =CH-$CH_3$, =CH-$CH_2$-$CH_3$, =N-NH-$CH_3$ ou = CH-S-$CH_3$
n représente un nombre de 0 à 10,
A représente hydrogène ou les restes alkyle, cycloalkyle, aryle ou hétaryle éventuellement substitués,
X, au cas où n = 0, est mis pour une liaison simple et au cas où n est différent de 0, est mis pour oxygène ou soufre ou une liaison simple, ainsi que leurs produits d'addition d'acide et produits d'addition de base tolérés par les plantes.

**7.** Insecticide caractérisé par le fait qu'il contient un véhicule inerte et une quantité efficace en tant qu'insecticide d'un composé de formule I

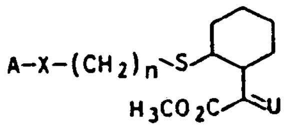

I

dans laquelle
U = $NOCH_3$, =$CHOCH_3$, =$CH_2$, =$CH$-$CH_3$, =$CH$-$CH_2$-$CH_3$, = N-NH-$CH_3$ ou = CH-S-$CH_3$
n représente un nombre de 0 à 10,
A représente hydrogène ou les restes alkyle, cycloalkyle, aryle ou hétaryle éventuellement substitués,
X, au cas où n = 0, est mis pour une liaison simple et au cas où n est différent de 0, est mis pour oxygène ou soufre ou une liaison simple, ainsi que leurs produits d'addition d'acide et produits d'addition de base tolérés par les plantes.

**8.** Procédé de lutte contre les champignons caractérisé par le fait que l'on traite les champignons ou les plantes, les semences, les matériaux ou le sol à protéger d'une attaque de champignons à l'aide d'une quantité efficace en tant que fongicide d'un composé de formule I

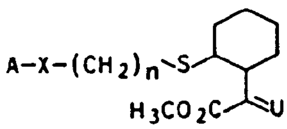

I

dans laquelle
U = $NOCH_3$, =$CHOCH_3$, =$CH_2$, =$CH$-$CH_3$, =$CH$-$CH_2$-$CH_3$, = N-NH-$CH_3$ ou = CH-S-$CH_3$
n représente un nombre de 0 à 10,
A représente hydrogène ou les restes alkyle, cycloalkyle, aryle ou hétaryle éventuellement substitués,
X, au cas où n = 0, est mis pour une liaison simple et au cas où n est différent de 0, est mis pour oxygène ou soufre ou une liaison simple, ainsi que leurs produits d'addition d'acide et produits d'addition de base tolérés par les plantes.